# EUROPEAN PATENT APPLICATION

(11) **EP 4 389 268 A1**
(43) Date of publication of application: **26.06.2024**
(21) Application number: 23217602.4
(22) Date of filing: 18.12.2023
(51) Int. Cl.: B01D 67/00, B01D 69/10, B01D 71/80

(54) **A METHOD FOR PRODUCING A BLOOD FILTER AND A BLOOD FILTER**

(30) Priority: 22.12.2022 EP 22216090
(71) Applicant: Imec VZW, 3001 Leuven (BE); Stichting IMEC Nederland, 5656 AE Eindhoven (NL)
(72) Inventor: CUMMINS, Cian, 3210 Lubbeek (BE); SEEMA SASEENDRAN, Sandeep, 3012 Wilsele (BE); HUMBERT, Aurelie, 1030 Schaarbeek (BE); WIERINGA, Fokko, 6661 RE Elst (NL); LINDEBOOM, Lucas, 5212 BT 's-Hertogenbosch (NL); LANGEREIS, Geert, 5611 HX Eindhoven (NL); VAN DEURSEN, Patrick, 1402 VK Bussum (NL)
(74) Representative: AWA Sweden AB

(57) **Abstract**

A method (100) for producing a blood filter (1), the blood filter (1) being a filter for filtering blood, the method (100) comprising
providing (S102) a first layer (30);
providing (S104) a block copolymer layer (40) above the first layer (30);
converting (S106) the block copolymer layer (40) to a mask (48) by selectively removing domains of the block copolymer layer (40);
etching (S108) pores (32) through the first layer (30) in regions exposed by the mask (48);
wherein a pore size is below 20 nm, the pore size preferably being 6.6 nm or smaller.

## Description

### TECHNICAL FIELD

The present inventive concept relates, in general, to a method for producing a blood filter and to a blood filter.

### BACKGROUND

A filter is a device that separates particles from a fluid. The filter may comprise a membrane, wherein pores extend through the membrane.

As an example, a filter may be a hollow fiber membrane, wherein hollow polymer fibers form the pores of the membrane. Such hollow polymer fibers may be produced by polymer extrusion through a spinneret.

As another example, a filter may be produced by etching holes through a solid membrane, wherein the holes form the pores of the membrane.

A blood filter is a filter for filtering blood. An example of a blood filter is a dialysis filter.

### SUMMARY

It is an objective of the present inventive concept to facilitate a blood filter of high quality. It is a further objective of the present inventive concept to facilitate a highly selective blood filter. It is a further objective of the present inventive concept to facilitate a blood filter with a high throughput. It is a further objective of the present inventive concept to facilitate a cheap blood filter. It is a further objective of the present inventive concept to facilitate blood filter that can be integrated with electronic circuits, in particular complementary metal-oxide-semiconductor (CMOS) circuits.

These and other objectives of the inventive concept are at least partly met by the invention as defined in the independent claims. Preferred embodiments are set out in the dependent claims.

According to a first aspect there is provided a method for producing a blood filter, the blood filter being a filter for filtering blood, the method comprising
providing a first layer;
providing a block copolymer layer above the first layer;
converting the block copolymer layer to a mask by selectively removing domains of the block copolymer layer;
etching pores through the first layer in regions exposed by the mask;
wherein a pore size is below 20 nm, the pore size preferably being 6.6 nm or smaller.

Blood passing through the pores may be filtered by the pores, when the blood filter is in use. Alternatively, the blood may be filtered by impurities of the blood being allowed to pass through the filter while at least some proteins of the blood are prevented from passing through the filter. Thus, the impurities may be removed from the blood such that the blood is filtered.

The first layer may comprise a semiconductor, an oxide or a nitride, e.g. Si, polycrystalline Si, SiO₂, Si₃N₄, or SiNx.

The block copolymer layer is a layer that comprises block copolymers (BCPs). A BCP may be a polymer comprising two or more polymer blocks, the two or more polymer blocks being different from each other. The polymer blocks may be homopolymer blocks. The polymer blocks may be linked by covalent bonds. The polymer blocks may form a linear chain. The BCPs may e.g. be diblock copolymers (di-BCPs), where each linear chain comprises two blocks, A and B. However, any type of BCP may be used, e.g. triblock copolymers (tri-BCPs).

Converting the BCP layer to a mask may comprise a self-assembly process. Herein, the BCPs may arrange to form domains, e.g. two or more types of domains. The formation of domains may be caused by the polymers of the different block types repelling each other, e.g. the polymer of block A repelling the polymer of block B. For example, chemically distinct homopolymers may repel each other. Thus, homopolymers of block A may be chemically distinct from homopolymers of the block B. Since the blocks are linked to each other, a microphase separation may occur in the BCP melt. After the microphase separation, the BCP layer may comprise A-type domains, comprising block A polymers, and B-type domains, comprising block B polymers. Various shapes and patterns are possible for the domains. Domains may be in the shape of cylinders (e.g. vertical cylinders in a hexagonally close-packed pattern), lamella (e.g. lamella in a pattern of curved paths), spheres (e.g. spheres in a body-centered cubic packed pattern), or gyroid networks. The self-assembly process may be initiated and/or controlled by e.g. baking the BCP layer. Baking may allow energy free minimum to be reached for the BCP. Baking, once above the BCP glass transition temperature, may introduce energy into the system that induces self-assembly. The baking temperature may control the shape and/or pattern of the domains. Baking may further solidify the BCP.

Converting the BCP layer to a mask further comprises selectively removing domains of the BCP layer. One domain type may e.g. selectively be removed by wet etch. The wet etch may herein be a solvent having a high solubility for one domain type and a low solubility for the other domain type. After etching, the first layer (or a hardmask layer in the form of e.g. a spin-on carbon layer and/or a spin-on glass layer) may be exposed in the regions of the selectively removed domains.

Exposed regions of the first layer are subsequently etched to form pores. The shape and pattern of the pores may herein be defined by the mask.

To exemplify, the conversion of the BCP layer to a mask may be done as follows. A BCP layer may be baked under conditions which induce cylindrical domains of block B polymers, B-type domains. Thus, these B-type domains are cylinders. Herein, the cylinders may extend perpendicular to the surface of the first layer. Such cylindrical domains may be termed vertical cylindrical domains. The B-type domains may then be selectively removed by a wet etch or dry etch. This may leave a layer comprising the A-type domains with cylindrical holes exposing a top surface of the first layer. Thus, a mask is formed such that the first layer may be etched in the exposed regions while the rest of the first layer is masked and therefore not etched.

The method facilitates production of a filter with a small membrane thickness, a small pore size, a highly controllable pore size, a uniform pore size distribution, a high pore density, a large membrane area and a high strength. Such filters may provide efficient and highly selective filtration with a high throughput. Such filters may be used as blood filters and are advantageous in many applications.

The method may be configured to form pores with a pore size below 20 nm by the etching. Alternatively, the method may be configured to form pores with a pore size below 20 nm by the etching followed by pore shrinking. Thus, as will be discussed below, pores with a pore size larger than 20 nm may be formed by the etching, after which the pores are shrunk by e.g. deposition of a polymer brush layer.

When filtering blood, the pore size may need to be small, as many components of blood are small. It may be advantageous with a pore size below 20 nm, such as a pore size below 10 nm, a pore size below 7 nm or a pore size of 6.6 nm or smaller. For example, the pore size may be in the range of 5-20 nm or in the range of 5-10 nm. The blood filter may then keep blood components that are larger than the pore size on one side of the filter while letting components of the blood that are smaller than the pore size pass to the other side. For example, proteins in the blood may be in the above mentioned size ranges and may be prevented from passing the blood filter. In contrast, smaller components, such as e.g. e.g. urea and creatinine, may pass. In particular, the blood filter may be configured to prevent albumin proteins from passing.

The pore size may be a lateral distance across the pore, i.e. a lateral distance across the pore in a direction parallel with the first layer. The pore size may be the smallest lateral distance across the pore. As an example, for a pore in the shape of a round hole, the pore size may be the diameter of the round hole. As another example, for a pore in the shape of a slit, the pore size may be the width of the slit. If the pore tapers from one side of the first layer to another side of the first layer, the pore size may be defined at the side where the pore is the most narrow.

As a first example of an application, the blood filter may be used as a dialysis filter, e.g. a kidney dialysis filter such as a hemodialysis filter, for purifying blood. It is a realization that the method according to the first aspect facilitate production of a blood filters with a thinner membrane, smaller pore sizes, and more uniform pore size distribution than a traditional hemodialysis filter. In a traditional hemodialysis filter, hollow polymer fibers form the pores of the membrane. A traditional hemodialysis filter generally comprises a membrane which can be as thick as several microns. A traditional hemodialysis filter generally has tortuous pores in a diffuse Gaussian size distribution, and a limited fill-factor (open pore area per area unit of the membrane) thereby slowing average diffusion time. This leads to the need for a high-power blood pump to drive the filter process, while blood filtration remains relatively ineffective. Also, either toxins near the size of albumin remain in a patient's blood (with smaller molecular size cut-off membranes), or loss of albumin and other critical proteins occur (with larger molecular size cut-off membranes). It is a realization that both scenarios are highly undesirable, and that a very steep size cut-off is advantageous. This can be achieved with highly uniformly sized pores, as advantageously provided by the method according to the inventive concept. In particular, a pore size of 6.6 nm is just below the size of albumin molecules and advantageously prevents albumin from passing.

When the blood filter according to the invention is used as a hemodialysis filter, the blood filter may be arranged with impure blood on a first side of the first layer and a dialysis solution on a second side of the first layer. Some components of the blood, like albumin molecules and/or other protein molecules, may then be too large to pass through the pores of the first layer while other components of the blood (like impurities such as urea, potassium, and phosphate, as well as excess water) may diffuse into the dialysis solution. Thereby, the impure blood may be purified.

As a second example, the blood filter may be used in conjunction with a sensor to be implanted in the body of a human or animal. The blood filter may be arranged as a semi permeable membrane over the sensor. The blood filter may be configured to prevent large components of the blood from reaching the sensor while smaller components, such as e.g. ions, may reach the sensor and be measured. Thus, the blood filter may prevent the sensor being rejected by the immune system of the body. The outer side of the blood filter, i.e. the side facing away from the sensor, may herein have a surface configured to be accepted by an immune system.

As a third example, the blood filter may be used as part of a lab-on-a chip system, e.g. together with a sensor in a lab-on-a chip system.

It is a realization that the use of a mask made from a BCP layer facilitates production of very small pores. Thus, an efficient filter may be produced. Such a filter may filter out most particles and only let the smallest particles pass. A mask made from a BCP layer facilitates production of pores with a size below 20 nm.

Further, a mask made from a BCP layer facilitates a uniform size distribution of the pores. For example, the variance of the pore size (e.g. the pore diameter) may be small when the pores are produced using a mask made from a BCP layer. Thus, highly selective filtration is facilitated. Phrased differently, the filter may have a steep size cut-off.

Further, a mask made from a BCP layer facilitates a high pore density. This allows a much smaller device footprint for the same filter function as compared to present filters. Fabricating compact, e.g. compact and ultrathin, membrane layers may facilitate devices moving beyond the current commercial membranes.

Further, a mask made from a BCP layer facilitates patterning of a large area of the first layer. The pattern of the mask may extend over the entire first layer. The membrane area and position may then be set by the use of a second mask, e.g. a hard mask under, or over, the BCP layer. Thus, large membranes may be produced, thereby facilitating a large flow through the filter.

Further, the size of the pores may be accurately controlled, e.g. by selecting the BCP composition and/or selecting the BCP molecular weight, and/or selecting the baking temperature. Further, the pattern of the pores may be accurately controlled, e.g. by selecting the BCP composition and/or selecting the BCP molecular weight, and/or selecting the baking temperature

Further, the filter may be produced in a cost-effective way. BCP patterns may have features so small that they otherwise would require costly lithographic techniques such as e.g. electron beam lithography. Such lithographic techniques may also be incompatible with CMOS processing at standard CMOS fabrication plants. Building electronic elements together with a long-term use filter using semiconductor fabrication methods can realize a rapid and low-cost strategy for e.g. artificial kidney devices. It is a realization that classical dialyzer membranes, e.g. comprising hollow polymer fibers, do not lend themselves to designing wearable and implantable devices. The CMOS process may produce an ultrathin and lightweight solution akin to today's state of the art high performance electronic devices.

The method may further comprise
providing a substrate before providing the first layer, such that the first layer is provided above the substrate;
forming a channel through the substrate, the channel being configured to provide fluid communication between a first and a second end of the channel, the first end of the channel being directly below the etched pores of the first layer,
whereby components of blood pass both the pores and the channel, when the filter is in use.

The substrate may comprise a semiconductor, e.g. a semiconductor wafer or a semiconductor layer. The semiconductor may be e.g. silicon or any other semiconductor. The substrate may comprise further layers above the semiconductor, e.g. an oxide layer above the semiconductor. To exemplify, the substrate may be e.g. a silicon wafer with an oxide layer on top, or just a silicon wafer. A wafer with further layers may be referred to as stack.

The channel formed through the substrate may extend to a bottom surface of the first layer. Thus, in the finished filter, blood components smaller than the pore size may flow from the top surface of the first layer, via the pores to the first end of the channel at the bottom surface of the first layer, and further to the second end of the channel. Blood components larger than the pore size may herein be prevented from entering the channel. It should be understood that the flow may alternatively be in the opposite direction, in this case blood components larger than the pore size may herein be prevented from leaving the channel.

The channel may extend through the substrate in a direction orthogonal to the first layer, wherein the second end of the channel is arranged at a bottom side of the substrate. Such a channel may be termed a vertical channel. The vertical channel may be formed by backside etching of the substrate. Herein, a resist may be deposited on the bottom side (i.e. backside) of the substrate. The resist may be patterned by lithography, e.g. with standard photolithography. The resist may subsequently be developed after which etching may form the vertical channel through the substrate to pores of the first layer. Dry etching may be used, e.g. deep reactive ion etching. Alternatively, or additionally, wet etching may be used, e.g. wet etching with potassium hydroxide KOH. Alternatively, or additionally, laser ablation may be used.

As an alternative, the channel may extend along an interface between the substrate and the first layer, wherein the second end of the channel is arranged at an opening in the first layer. Such a channel may be termed a horizontal channel. The horizontal channel may be formed in a buried oxide layer (BOX layer) arranged below the first layer. The opening in the first layer may be formed next to the pores of the first layer, e.g. at a distance from the pores of the first layer. The horizontal channel may then be formed by etching, e.g. wet etching or dry etching, the horizontal channel between the opening in the first layer and the pores of the first layer. Thus, the horizontal channel may extend underneath, and in parallel with, the first layer.

A vertical channel advantageously facilitates a laminar flow of the fluid. A horizontal channel advantageously facilitates a cheap and/or fast production method. For example, wet etching a relatively short channel, e.g. 1-100 µm long or 1-10 µm long, underneath the first layer may be cheaper and/or faster than etching through a thick substrate.

The method may comprise a step of shrinking the pores. In the following, three different ways to shrink the pores will be discussed: pore shrinking using a polymer brush layer, pore shrinking using a film on inner surfaces of the pores, and pore shrinking using oxidation of inner surfaces of the pores. Thus, even smaller pores may be provided. The use of pore shrinking may provide a highly controllable pore size and/or a uniform pore size distribution. For example, consider the following two alternatives. In alternative 1, a BCP mask is used to define a small pore size. In alternative 2, a BCP mask is used to define a slightly larger pore size and the pores are subsequently shrunk by one of the three pore shrinking methods. At least under some conditions, e.g. for very small pore sizes, alternative 2 may provide a more controllable pore size and/or uniform pore size distribution.

Firstly, pore shrinking using a polymer brush layer will be discussed. The method may accordingly comprise:
removing the mask; and
forming a polymer brush layer above the first layer after removal of the mask, the polymer brush layer being a polymer layer comprising a plurality of polymer chains wherein each polymer chain is attached to an underlying layer at one end of the polymer chain.

The polymer brush layer may be formed directly on top of the first layer or formed such that there is a layer in between the first layer and the polymer brush layer. The polymer brush layer may be formed directly on the layer that has been etched to form the pores. Alternatively, another layer may be deposited after etching of the pores but before the formation of the polymer brush layer. The polymer brush layer may be formed by deposition via e.g. spin coating or polymerization. The polymer brush layer may comprise polyethylene. Alternatively, or additionally, the polymer brush layer may comprise carboxybetaine (CB) moieties and/or photosensitive cross-linking moieties. The latter two may, due to their stability, be particularly useful for filters that are to be used inside the body of a person or animal. To further exemplify, the polymer brush layer may comprise poly(carboxybetaine) and/or zwitterionic poly(carboxybetaine).

The polymer brush layer may shrink the pores. Polymer chains in the vicinity of a pore may extend, with the unattached end, over the edge of the pore. This may happen around the circumference of the pore. Thereby the pore size may be reduced. Further, the polymer brush layer may provide an antifouling surface. Thus, the polymer brush layer may resist adsorption of proteins and/or cells and/or bacteria.

The method may further comprise:
providing an oxide layer above the first layer before providing the block copolymer layer; and
wherein the polymer brush layer is formed above the oxide layer such that the polymer chains of the polymer brush layer are attached to the first layer by the oxide layer.

Thus, said oxide layer may function both as a hard mask for etching the pores and also enhance adhesion of the polymer brush layer. The pattern of the mask may be transferred to the oxide layer, e.g. by etching. For example, the oxide layer may be wet etched in the regions exposed by the mask. The pores may then be dry etched, wherein the oxide layer functions as a hard mask. During said dry etch, some (or all) of the BCP mask may disappear. Remaining BCP mask may then be removed, e.g. dissolved. Subsequently, the polymer brush layer may be deposited. The oxide layer may then provide hydroxyl (-OH) groups at the surface. The -OH groups may enable covalent bonding to the polymer brush layer or allow radical polymerisation of the polymer brush layer to occur.

Secondly, pore shrinking by use of a film on inner surfaces of the pores will be discussed. The method may accordingly comprise:
depositing a film on inner surfaces of the pores of the first layer.

Such a film on inner surfaces of the pores may shrink the pore size. The film on inner surfaces of the pores may be deposited by a conformal deposition technique. The film may be deposited by atomic layer deposition (ALD) and/or chemical vapor deposition and/or self-assembly of monolayers and/or metal ion infusion using polymer brushes and/or sputtering. ALD may be particularly advantageous. ALD may provide highly conformal deposition. Additionally, or alternatively, ALD may provide deposition with high control of the thickness of the deposited material. The film on inner surfaces of the pores may comprise e.g. aluminum oxide or hafnium oxide.

Thirdly, pore shrinking by use of oxidized inner surfaces of the pores will be discussed. The method may accordingly comprise:
oxidizing inner surfaces of the pores of the first layer.

Such oxidized inner surfaces of the pores may shrink the pore size. Oxidation of material of the first layer on the inner surfaces of the pores may cause layer growth which shrinks the pore size.

As previously mentioned, various shapes and patterns are possible for the domains of the BCP layer. Cylindrical domains and lamella domains are particularly useful for the method. The formation of filters by use of such domains will be discussed in the following.

The method may be configured for production of round hole pores:
wherein the block copolymer layer comprises vertical cylindrical domains, each vertical cylindrical domain being a cylindrical domain with an orientation perpendicular to the first layer; and
wherein the block copolymer layer is converted to a mask by selectively removing the vertical cylindrical domains of the block copolymer layer,
whereby the pores of the first layer comprise a plurality of round holes.

As previously mentioned, vertical cylindrical domains of the BCP layer extend perpendicular to the surface of the first layer. Thus, a cylinder axis of a vertical cylindrical domain may be perpendicular to the first layer. After removal of the vertical cylindrical domains, the BCP layer may comprise cylindrical holes exposing a top surface of the first layer. Thus, a mask is formed such that the first layer may be etched in the exposed regions while the rest of the first layer is masked and therefore not etched. The etched pores may then have a round cross-section. The etched pores may be cylindrical, e.g. have the same cylindrical shape as the vertical cylindrical domains. However, it should be understood that the formation of tapered pores is also possible, e.g. if the etch is isotropic. Such pores may have the shape of a truncated cone.

The vertical cylindrical domains of the BCP layer may arrange, or be compelled to arrange, as a lattice. The lattice may e.g. be a close packed lattice.

The method may be configured for production of slit pores:
wherein the block copolymer layer comprises lamella domains or horizontal cylindrical domains, each horizontal cylindrical domain being a cylindrical domain with an orientation parallel with the first layer; and
wherein the block copolymer layer is converted to a mask by selectively removing the lamella domains or horizontal cylindrical domains of the block copolymer layer,
whereby the pores of the first layer comprise a plurality of slits.

As a first example, the method may be configured for production of slit pores:
wherein the block copolymer layer comprises lamella domains; and
wherein the block copolymer layer is converted to a mask by selectively removing the lamella domains of the block copolymer layer,
whereby the pores of the first layer comprise a plurality of slits.

A lamella domain may be a domain shaped like a wall, wherein the wall extends along a curved, or straight, path across the surface of the first layer. A lamella domain may be a domain with a rectangular (or trapezoid) cross-section, wherein the lamella domain extends along a curved, or straight, path across the surface of the first layer.

A lamella domain extending along a curved path across the surface of the first layer may be particularly advantageous. Such a lamella domain may form a slit extending along a curved path across the surface of the first layer. This may form a "fingerprint" like pattern of curved slits. Slits extending along curved paths and advantages thereof will be discussed further below.

As a second example, the method may be configured for production of slit pores:
wherein the block copolymer layer comprises horizontal cylindrical domains, each horizontal cylindrical domain being a cylindrical domain with an orientation parallel with the first layer; and
wherein the block copolymer layer is converted to a mask by selectively removing the horizontal cylindrical domains of the block copolymer layer,
whereby the pores of the first layer comprise a plurality of slits.

Horizontal cylindrical domains of the BCP layer extend in parallel with the surface of the first layer. Thus, a cylinder axis of a horizontal cylindrical domain may be in parallel with the first layer. A horizontal cylindrical domain may be a cylinder lying on the surface of the first layer.

A horizontal cylindrical domain may extend along a curved, or straight, path across the surface of the first layer.

Horizontal cylindrical domains may be formed from an asymmetric BCP system. Similarly, vertical cylindrical domains may be formed from an asymmetric BCP system. The orientation of the cylindrical domains (vertical or horizontal) may be set e.g. by the thickness of the BCP layer and/or by annealing conditions.

The method may facilitate production of a filter with a fast transmembrane diffusion speed. Thus, fluid may pass through the filter rapidly. A fast transmembrane diffusion speed may be facilitated in many different ways.

As an example, the method may facilitate production of a filter with a thin membrane. A thin membrane may facilitate a fast transmembrane diffusion speed as fluid does not have to travel a long distance through the pores of the membrane.

The first layer may e.g. be 20 nm to 2000 nm thick, this may result in a membrane which is 20 nm to 2000 nm thick.

The first layer may e.g. be 20-500 nm thick. Membranes in the range 20-500 nm may be durable enough to be implanted in a body. Membranes in the range 20-500 may also be suitable for hemodialysis outside the body.

As another example, the method may facilitate production of a filter wherein a fill-factor of the pores is at least 40%, the fill-factor of the pores being open pore area per area unit of the first layer. The fill-factor may e.g. be at least 50%.

Thus, the fill-factor of the pores may be the area of the pores divided by the sum of the area of the pores and the area between the pores. The area of the pores and the area between the pores may herein be areas within the plane of the first layer. The area of the pores may correspond to an area of the membrane of the filter through which fluid can flow. The area between the pores may correspond to an area of the membrane of the filter through which fluid cannot flow.

Thus, the method facilitates production of a filter with a high fill-factor. This may lead to a low filter resistance. The filter resistance may herein be defined as the pressure drop across the filter at a stated flow. The filter resistance is generally expressed in millimeters water gauge or PSI, or in SI units as N/m² or Pascals.

A filter with a high fill-factor of the pores, i.e. a high porosity, i.e. a low filter resistance may be made thin without breaking (since there is not a large pressure drop over the filter), thus the membrane facilitates a fast transmembrane diffusion speed.

A filter with a high fill-factor of the pores, i.e. a high porosity may further sustain a large flow even if the membrane is small. Thus, the footprint of the filter may be small.

A filter with a high fill-factor of the pores, i.e. a high porosity may further be strong. In view of the above, such a filter may be subjected to a small pressure drop across the membrane, whereby the membrane may be subjected to smaller forces. In view of the above, such a filter may be made smaller and thereby more durable.

As another example, the method may facilitate production of a filter with tapered pores. Thus, the method may comprise:
shaping the pores of the first layer such that the pores taper.

A tapered pore may have a wide end and a narrow end.
Tapered pores may facilitate highly selective filtration with a low filter resistance. The selectivity may herein be ensured by the narrow end of the pore. Particles larger than the narrow end of the pore may be prevented from passing the pore. However, the filter resistance may still be low as the wide end of the pore restricts the flow of fluid less than it would if it had the same size as the narrow end. When used, the narrow end of the pores may face the fluid to be filtered.

A filter with tapered pores, i.e. having a low filter resistance, may be made thin without breaking (since there is not a large pressure drop over the filter), thus the membrane facilitates a fast transmembrane diffusion speed.

A filter with tapered pores, i.e. having a low filter resistance, may further sustain a large flow even if the membrane is small. Thus, the footprint of the filter may be small.

A filter with tapered pores, i.e. having a low filter resistance, may further be strong. In view of the above, such a filter may be subjected to a small pressure drop across the membrane, whereby the membrane may be subjected to smaller forces. In view of the above, such a filter may be more durable.

As understood from the above, a thin first layer and/or a high fill-factor and/or tapered pores may facilitate miniaturization of the blood filter, increases portability and make implantability more feasible. Fabricating compact and ultrathin membrane layers facilitates wearable and implantable devices.

An advantage of the method is that it facilitates production of a filter with integrated electronics. For example, the substrate may comprise one or more integrated circuits. Thus, the method may comprise the step of providing a substrate, wherein the substrate comprises an integrated circuit. Alternatively, the method may comprise the step of forming an integrated circuit in or on the substrate.

The electrical circuit may be a CMOS circuit as the method is CMOS compatible. The method may e.g. be performed at a standard CMOS fabrication plant. In particular, it is a realization that present filter fabrication methods which could be applied at a standard CMOS fabrication plant would also have inherent restrictions to the maximum reachable pore density and/or to the minimum pore size. Such restrictions may be overcome by use of the method of the first aspect.

The integrated circuit may be a sensor, an actuator, a processor, a memory, an antenna structure (e.g. antenna structure for generation or detection of electromagnetic fields and/or wireless communication and/or power management).

Since the method facilitates production of a filter with a small footprint, there may be more room for a larger and/or more advanced integrated circuit. A filter wherein the substrate comprises one or more integrated circuits and wherein the fill-factor of the pores is above 40% is particularly advantageous.

It is a realization that it may be particularly advantageous when the block copolymer layer comprises:
polystyrene-polymethylmethacrylate block copolymers, and/or
polylactic acid-polyvinylpyridine block copolymers, and/or
polyethylene oxide-polydimethylsiloxane block copolymers.

Such BCPs may be particularly useful for producing membranes with production of a filter with a small pore size and/or a highly controllable pore size and/or uniform pore size distribution and/or a high pore fill-factor.

It is a realization that it may be particularly advantageous to use a block copolymer layer with a high chi, e.g. chi being above 0.3.

The block copolymer layer may comprise block copolymers with a molecular weight below 30 kg/mol, such as below 20 kg/mol or in the range of 10-20 kg/mol.

Again, such BCPs may be particularly useful for producing membranes with production of a filter with a small pore size and/or a highly controllable pore size and/or uniform pore size distribution and/or a high pore fill-factor.

According to a second aspect, there is provided a blood filter comprising:
a first layer;
pores extending through the first layer, wherein a pore size is below 20 nm, the pore size preferably being 6.6 nm;
wherein the pores of the first layer comprises a plurality of round holes and wherein the plurality of round holes are arranged as a first lattice in a first region of the first layer and as a second lattice in a second region of the first layer, the first and second lattice having same periodicity but different orientations, or
wherein the pores of the first layer comprise a plurality of slits, and wherein each slit of the plurality of slits extends along a curved path across a surface of the first layer.

A filter according to the second aspect may have the same advantages, or similar advantages, as the advantages described in conjunction with the first aspect.

When applying the method according to the first aspect and using a BCP layer comprising vertical cylindrical domains, the result may be a filter wherein the pores of the first layer comprise a plurality of round holes and wherein the plurality of round holes are arranged as a first lattice in a first region of the first layer and as a second lattice in a second region of the first layer, the first and second lattice having same periodicity but different orientations. This may be the case at least under conditions where the membrane is large and/or where a guiding pattern is not used to compel the vertical cylindrical domains to adopt the same lattice over a large area. Thus, the lattice constant and/or lattice type may be the same for the first and second lattice. The second lattice may be rotated, e.g. rotated around a normal to the first layer, in relation to the first lattice. The second lattice may be rotated by at least 5°, such as rotated by at least 20°. The first and second regions may, respectively, have areas of at least 10 µm², such as areas of at least 100 µm².

When applying the method according to the first aspect and using a BCP layer comprising lamella domains, the result may be a filter wherein the pores of the first layer comprise a plurality of slits, and wherein each slit of the plurality of slits extends along a curved path across a surface of the first layer. This may be the case at least under conditions where the membrane is large and/or where a guiding pattern is not used to compel the lamella domains to follow straight paths over a large area. A curved path of a slit may be a path that within at least one segment of the path has a radius of curvature smaller than 1 mm, such as a radius of curvature smaller than 10 µm. The above applies also to the case when a BCP layer comprises horizontal cylindrical domains. Horizontal cylindrical domains may be similar to lamella domains except for the cross section of a horizontal cylindrical domain being circular instead of rectangular.

The filter may further be strong when the round holes are arranged as a first lattice in a first region of the first layer and as a second lattice in a second region of the first layer. A lattice may have certain cleavage directions or cleavage planes, e.g. along a row of round holes. Such cleavage directions may be seen as directions which are prone to crack formation. When the membrane of the filter comprises two regions which have different lattice orientations, the first region may resist crack formation in a direction in which the second region is prone to crack formation, and vice versa.

Similarly, the filter may be strong when the pores of the first layer comprise a plurality of slits which extend along curved paths. The curves may herein reduce periodicity of the slits and thereby resist crack formation.

### BRIEF DESCRIPTION OF THE DRAWINGS

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 illustrates a flowchart of a method
Fig. 2 illustrates production of round hole pores
Fig. 3 illustrates production of slit pores
Fig. 4 illustrates a filter with a vertical channel
Fig. 5 illustrates a filter with a horizontal channel
Fig. 6 illustrates tapered round hole pores
Fig. 7 illustrates tapered slit pores
Fig. 8 illustrates pore shrinking using a polymer brush layer
Fig. 9 illustrates pore shrinking using a film on inner surfaces of pores
Fig. 10 illustrates pore shrinking using oxidized inner surfaces of pores
Fig. 11 illustrates a flowchart of a method
Fig. 12 illustrates steps of a method
Fig. 13 illustrates a filter
Fig. 14 illustrates a filter

### DETAILED DESCRIPTION

In cooperation with attached drawings, the technical contents and detailed description of the present invention are described thereinafter according to a preferable embodiment, being not used to limit the claimed scope. This invention may be embodied in many different forms and should not be construed as limited to the embodiments set forth herein; rather, these embodiments are provided for thoroughness and completeness, and fully convey the scope of the invention to the skilled person.

In the following, methods for producing filters and filters will be discussed. The filters discussed may be used as blood filters.

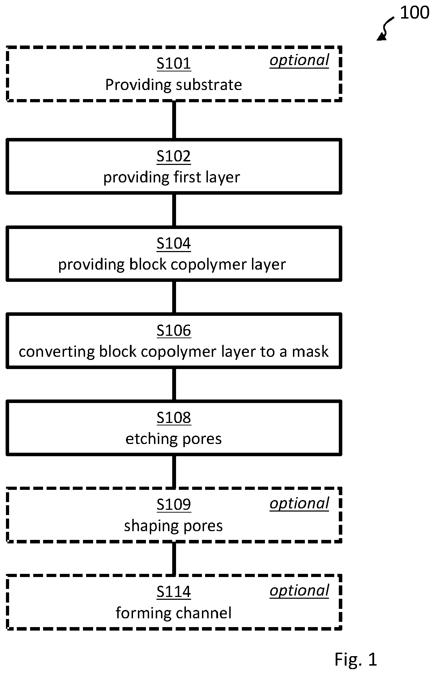

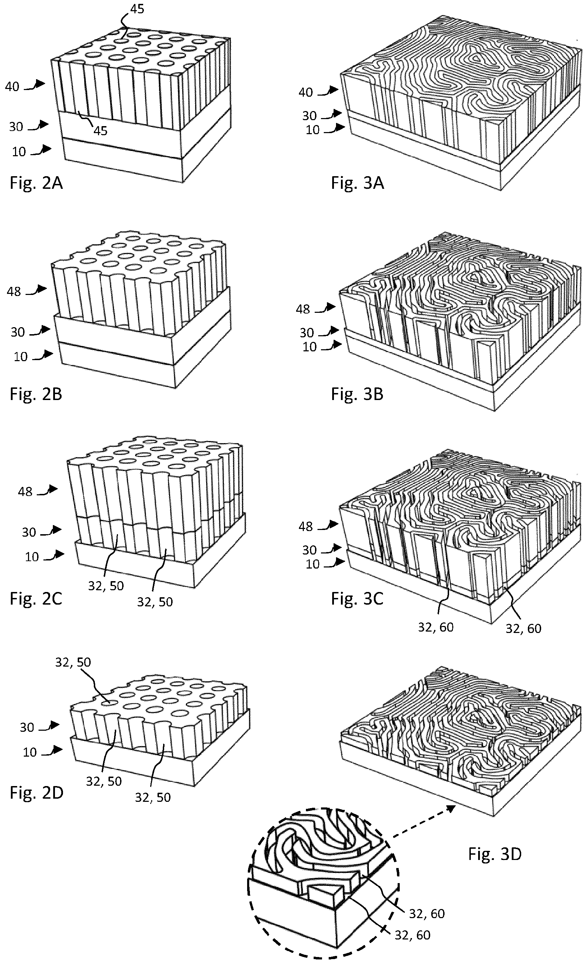

Fig. 1 illustrates a flowchart of a method 100 for producing a filter 1. The method 100 may be used to produce a filter 1 with pores 32 in the shape of round holes 50 or in the shape of slits 60. The method 100 illustrated in Fig. 1 will hereinafter be discussed together with Figs 2A-D and Figs 3A-D. Figs 2A-D illustrate cut-outs of the filter 1 during the production of round hole pores 32, 50. Figs 3A-D illustrate cut-outs of the filter 1 during the production of slit pores 32, 60.

The method 100 comprises the optional step of providing S101 a substrate 10. The method 100 comprises the steps of providing S102 a first layer 30, e.g. above the substrate 10, and providing S104 a block copolymer layer 40 above the first layer 30.

In the following, the method will be described as the first layer 30 being provided on a substrate 10. However, it should be understood that the method may also be applied in situations where a substrate 10 is not used.

The substrate 10 may e.g. be a stack comprising a wafer 12 and an oxide layer 14 on top of the wafer 12. The first layer 30 may comprise a semiconductor, an oxide or a nitride, e.g. Si, SiO₂, Si₃N₄. The block copolymer layer 40 may comprise polystyrene-polymethylmethacrylate (polystyrene-PMMA) block copolymers, and/or polylactic acid-polyvinylpyridine block copolymers, and/or polyethylene oxide-polydimethylsiloxane block copolymers.

Fig. 2A illustrates a cut-out of a filter 1 (during production) after a BCP layer 40 has been provided. The BCP layer 40 in Fig. 2A comprises vertical cylindrical domains 45. Such vertical cylindrical domains 45 may be formed e.g. when the BCP layer 40 comprises polystyrene-b-PMMA which has been baked at 250 °C. The vertical cylindrical domains 45 may be configured to have diameter smaller than 100 nm, such as smaller than 20 nm. The vertical cylindrical domains 45 may be configured to have a fill-factor of at least 40%.

Fig. 3A illustrates a cut-out of a filter 1 (during production) after a BCP layer 40 has been provided. The BCP layer 40 in Fig. 2A comprises lamella domains 46. Such lamella domains 46 may be formed e.g. when the BCP layer 40 comprises polystyrene-b-PMMA which has been baked at 250 °C. The lamella domains 46 may be configured to have a width smaller than 100 nm, such as smaller than 20 nm. The lamella domains 46 may be configured to have a fill-factor of at least 40%. As understood by the skilled person, the lamella domains 46 may be replaced by horizontal cylindrical domains.

The method 100 further comprises the step of converting S106 the block copolymer layer 40 to a mask 48 by selectively removing domains of the block copolymer layer 40.

Fig. 2B illustrates a cut-out of a filter 1 (during production) after the vertical cylindrical domains 45 have been removed. Thus, a mask 48 is formed where the first layer 30 may be exposed in the regions where the vertical cylindrical domains 45 previously were. Vertical cylindrical domains 45 of PMMA may be selectively removed from a polystyrene surrounding by a wet etch of acid acetic.

Fig. 3B illustrates a cut-out of a filter 1 (during production) after the lamella domains 46 have been removed. Thus, a mask 48 is formed where the first layer 30 may be exposed in the regions where the lamella domains 46 previously were. Lamella domains 46 of PMMA may be selectively removed from a polystyrene surrounding by a wet etch of acid acetic.

The method 100 further comprises the step of etching S108 pores 32 through the first layer 30 in regions exposed by the mask 48. For example, the pores may be formed by dry etching.

Fig. 2C illustrates a cut-out of a filter 1 (during production) after etching S108 of pores 32 through the first layer 30. The pores 32 herein have the shape of round holes 50. The pores 32 may have the same shape as the previous vertical cylindrical domains 45. Fig. 2D illustrates the round hole pores 32, 50 after removal of the mask 48.

Fig. 3C illustrates a cut-out of a filter 1 (during production) after etching S108 of pores 32 through the first layer 30. The pores 32 herein have the shape of slits 60. The pores 32 may have the same shape as the previous lamella domains 46. Fig. 3D illustrates the slit pores 32, 50 after removal of the mask 48. A close-up view of the slit pores 32, 50 is also presented in Fig. 3D.

The method 100 may further comprise the optional step of forming S114 a channel 20 through the substrate 10, the channel 20 being configured to provide fluid communication between a first 21 and a second 22 end of the channel 20, the first end 21 of the channel 20 being directly below the etched pores 32 of the first layer 30.

The channel 20 may extend through the substrate 10 in a direction orthogonal to the first layer 30, wherein the second end 22 of the channel 20 is arranged at a bottom side of the substrate 10. Such a channel may be termed a vertical channel. As an alternative, the channel 20 may extend along an interface between the substrate 10 and the first layer 30, wherein the second end 22 of the channel 20 is arranged at an opening 26 in the first layer. Such a channel 20 may be termed a horizontal channel.

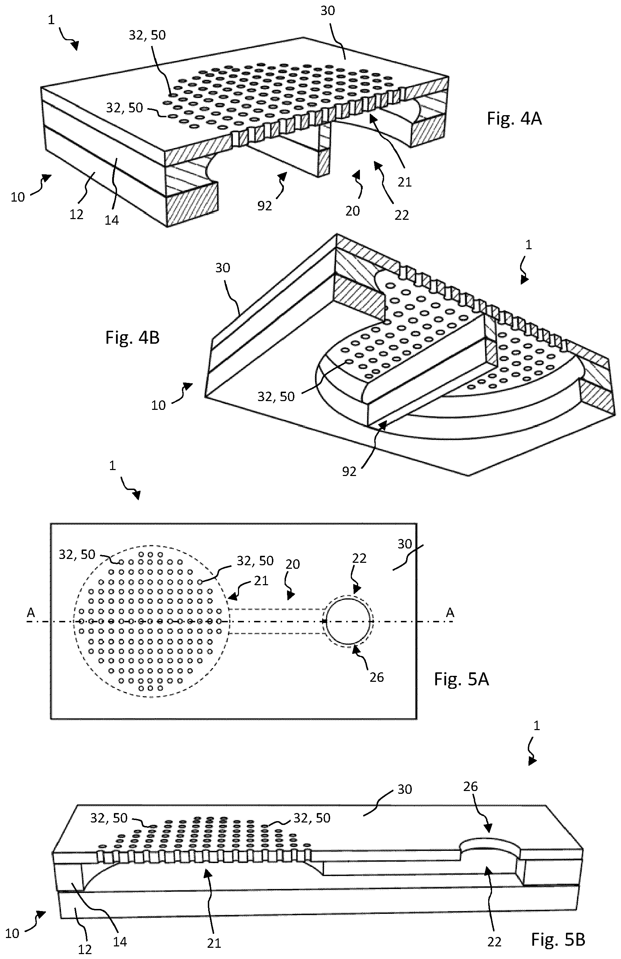

Fig. 4A illustrates a perspective view of a cross-section of a filter 1 with a vertical channel. Fig. 4B illustrates a perspective view of the same cross-section of the filter 1 of Fig. 4A, seen from the bottom side.

Fig. 5A illustrates a top view of a filter 1 with a horizontal channel. Fig. 5B illustrates a perspective view of a cross-section of the filter 1 of Fig. 5A. The cross-section being along the A-A line in Fig. 5A.

A vertical channel, such as the one in Figs 4A-B, may be formed by backside etching of the substrate. Dry etching may be used, e.g. deep reactive ion etching. Alternatively, or additionally, wet etching may be used, e.g. wet etching with potassium hydroxide KOH. Alternatively, or additionally, laser ablation may be used. In Figs 4A-B there is a support 92 for the membrane. The support may comprise a part of the substrate 10. Thus, the method may comprise forming a support 92 for the membrane. Thus, formation of the channel may be configured to leave part of the substrate 10 below the membrane such that a support 92 for the membrane is formed.

A horizontal channel, such as the one in Figs 5A-B, may be formed in a buried oxide layer (BOX layer) arranged below the first layer 30. The BOX layer may herein be an oxide layer 14 which is part of the substrate 10, as illustrated in Fig. 5B. The opening 26 in the first layer 30 may be formed next to the pores 32 of the first layer 30, e.g. at a distance from the pores 32 of the first layer 30. The opening 26 in the first layer 30 may be formed at a distance from the membrane.

The horizontal channel may then be formed by etching, e.g. wet etching, the horizontal channel between the opening in the first layer and the pores of the first layer. Thus, the horizontal channel may extend underneath, and in parallel with, the first layer, as illustrated in Figs 5A-B. Etch fluid may herein pass through the pores 32 and/or the opening 26 in the first layer 30 to form the horizontal channel. The dimensions and/or direction of the channel 20 may be controlled by e.g. doping selective etching. For example, in Figs 5A-B the BOX layer 14 in the region between the pores 32 and the opening 26 may be doped differently from the rest of the BOX layer 14 such that the etch selectively etches this region.

According to an embodiment, a protective layer may be deposited on the first layer 30 on a frontside of the filter 1 during etching from the backside.

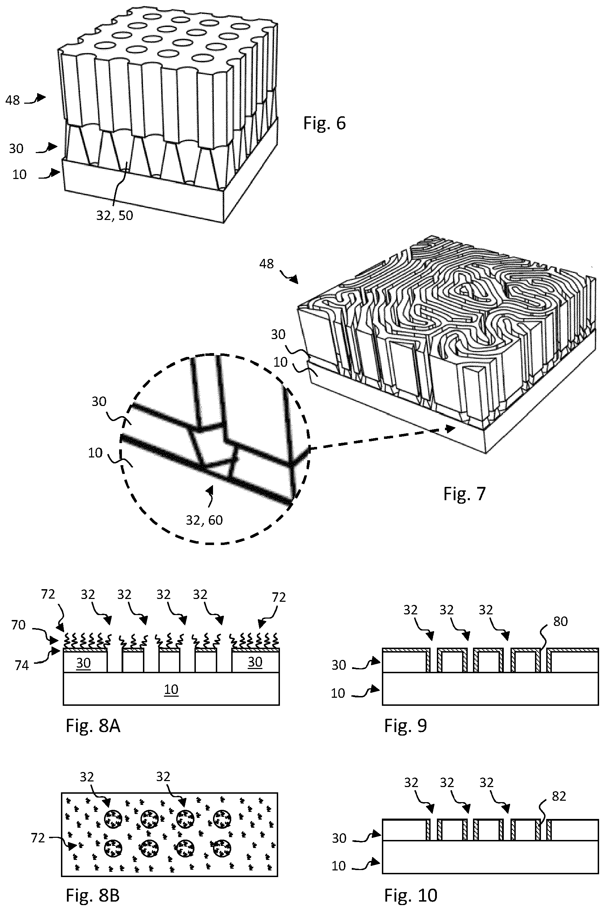

The method 100 may further comprise the optional step of shaping S109 the pores 32 of the first layer 30 such that the pores 32 taper. Fig. 6 illustrates tapered round hole pores 32, 50 in a cut-out of a filter 1 (during production). Fig. 7 illustrates tapered slit pores 32, 60 in a cut-out of a filter 1 (during production). A close-up view of one of the slit pores 32, 60 is also presented in Fig. 7.

The tapered pores 32 may, as illustrated, be wider at the top side of the first layer 30 than at the bottom side of the first layer 30. Thus, the diameter of a round hole pore 32, 50 may be larger at the top side of the first layer 30 than at the bottom side of the first layer 30. Similarly, the width of a slit pore 32, 60 may be larger at the top side of the first layer 30 than at the bottom side of the first layer 30.

Pores 32 of the first layer 30 may be shaped S109 to taper by undercut etching such that an undercut under the mask is formed. Undercut etching may be achieved by use of an isotropic etch.

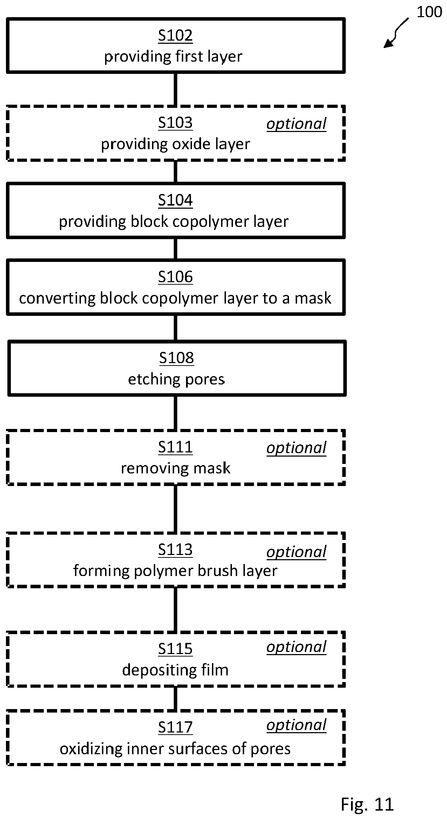

The method 100 may comprise the optional step of shrinking the pores 32. In the following, three different ways to shrink the pores 32 will be discussed: pore shrinking using a polymer brush layer 70, pore shrinking using a film 80 on inner surfaces of the pores 32, and pore shrinking using oxidated inner surfaces 82 of the pores. Fig. 11 illustrates a flow chart of a method 100 comprising optional steps for shrinking the pores.

Fig. 8A-B schematically illustrates how pores 32 may be shrunk using a polymer brush layer 70. Fig. 8A is a cross-sectional view of a filter 1 (during production, before forming the channel 20). Fig. 8B is a top view of the same filter 1. The polymer brush layer 70 comprises a plurality of polymer chains 72 wherein each polymer chain is attached to an underlying layer at one end of the polymer chain 72, as seen in Fig. 8A. Further, in Fig. 8A the polymer chains 72 are attached to an oxide layer 74, the oxide layer 74 being arranged on top of the first layer 30. As seen in Fig. 8A-B, polymer chains 72 in the vicinity of a pore 32 may extend, with the unattached end, over the edge of the pore 32. This may happen around the circumference of the pore 32. Thereby the pore size may be reduced. As seen in Fig. 11 the method may comprise the optional steps of removing S111 the mask 48 and thereafter forming S113 a polymer brush layer 70 above the first layer 30. If the polymer brush layer 70 is to be attached to an oxide layer 74, the method 100 may further comprise the step of providing S103 an oxide layer 74 above the first layer 30 before providing the BCP layer 40.

To exemplify the above: An oxide layer 74 of e.g. silicon oxide, e.g. 200 nm thick silicon oxide, may be provided on top of the first layer 30 before providing the BCP layer 40. After etching of the pores 30 and removal of the mask 48, a polymer brush layer 70 may be spin coated or polymerized on top of the oxide layer 74 such that the polymer chains 72 attach to the oxide layer 74. In accordance with the above, the oxide layer 74 may function as a hardmask during etching of the underlying layer, it may further increase the nanopore aspect ratio, and it may further act as a binding surface for the polymer brush layer 70.

Fig. 9 schematically illustrates how pores 32 may be shrunk using a film 80 on inner surfaces of pores 32. Fig. 9 is a cross-sectional view of a filter 1 (during production, before forming the channel 20). The illustrated film 80 conformally coats the surface of the first layer 30, including the inner surfaces of the pores 32. The film 80 may be deposited by atomic layer deposition (ALD) and/or chemical vapor deposition and/or self-assembly of monolayers and/or metal ion infusion using polymer brushes and/or sputtering. The film 80 may be deposited before or after the formation of the channel 20. If the film 80 is deposited before the formation of the channel 20, parts of the film 80 at the bottom of the pores 32 may be removed by etching. In accordance with the above, and as seen in Fig. 11, the method may comprise the optional step of depositing S115 a film 80 on inner surfaces of the pores 32 of the first layer 30.

Fig. 10 schematically illustrates how pores 32 may be shrunk using oxidized inner surfaces 82 of pores 32. The inner surfaces of the pores may be oxidized during etching of the pores 32 or in a subsequent oxidation step. The inner surfaces of the pores 32 may be oxidized by thermal oxidation, e.g. by dry or wet thermal oxidation. In accordance with the above, and as seen in Fig. 11, the method may comprise the optional step of oxidizing S117 inner surfaces of the pores 32 of the first layer 30.

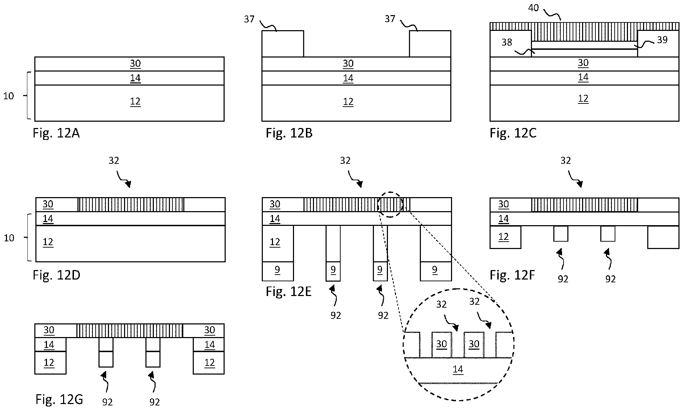

In the following an example of a production flow for a filter 1 with a vertical channel 20 is given. The production flow is illustrated by Figs 12A-G which show the filter (during production) in cross-sectional views.

A silicon wafer 12 with an oxide layer 14 on top forms the substrate 10. The silicon wafer may be 450-600 µm thick. The oxide layer 14 may be e.g. 2000 nm thick. A first layer 30 of Si, polycrystalline Si, SiO₂, Si₃N₄, or SiNx may be provided on top of the oxide layer 14, see Fig. 12A. The thickness of the first layer 30 may be e.g. 20-2000 nm, preferably 20-500 nm. As shown in Fig. 12B, a select area of the first layer 30 may be opened by conventional photolithography patterning of a hardmask 37, the hardmask 37 may comprise a dielectric (Al₂O₃) or metal (TaN, Ru etc.). This process can be achieved by dry or wet etch means.

As seen in Fig. 12C, a spin on carbon layer 38 and spin on glass layer 39 may be deposited, followed by a BCP layer 40. The BCP layer 40 may be baked to self-assemble into suitable domains. The domains may be vertical cylindrical domains 45 with a diameter smaller than 100 nm, such as smaller than 20 nm. The domains may be lamella domains 46 with a width smaller than 100 nm, such as smaller than 20 nm.

The BCP layer 40 may then be converted to a mask 48. The spin on carbon layer 38 and/or the spin on glass layer 39 may further be converted to part of the mask 48.

Selective dry etching may then be used to transfer the pattern of the mask 48 to the underlying first layer 30, as seen in Fig. 12D. The hardmask 37 may also be removed, as further seen in Fig. 12D.

Next, a backside resist 9 may be deposited and patterned with standard photolithography. The backside resist 9 may subsequently be developed after which dry etching leads to part of the channel 20 being formed, e.g. the part up to the oxide layer 14, see Fig. 12E. Part of the substrate 10 may be left to form a support 92 for the membrane.

The backside resist may subsequently be removed and supports 92 may be thinned down, e.g. to a height of 50-100 µm, see Fig. 12F.

Finally, the exposed oxide layer 14 may be wet etched to release the first layer 30 which then forms the membrane layer in the released region.

According to an embodiment, the final release of the membrane can be achieved through wet etching (e.g., buffered hydrogen fluoride (HF)) or dry etching (e.g., plasma, vapor HF).

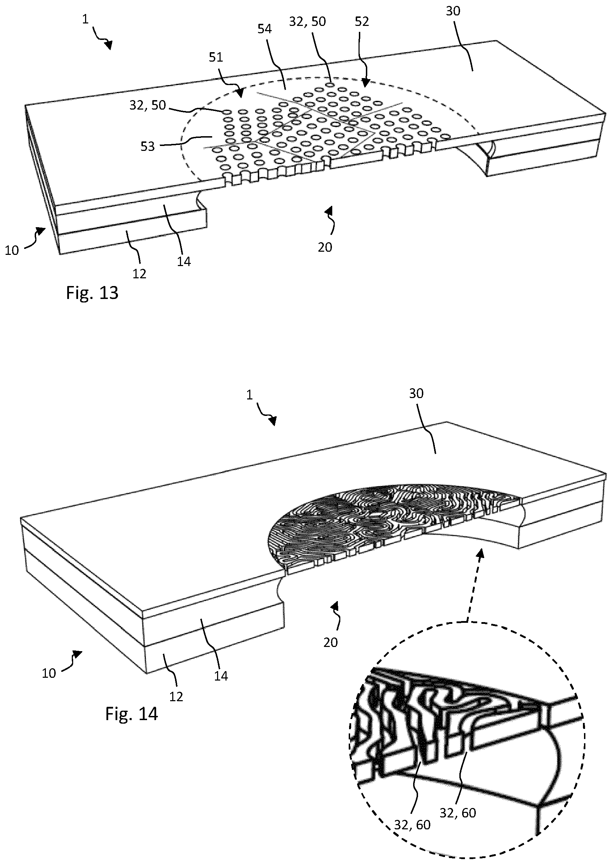

Figs 13 and 14 illustrate two different types of filters 1 which may be produced by the method 100.

Fig. 13 illustrates a perspective view of a cross-section of a filter 1 wherein the pores 32 of the first layer 30 comprise a plurality of round holes 50 and wherein the plurality of round holes 50 are arranged as a first lattice 51 in a first region 53 of the first layer 30 and as a second lattice 52 in a second region 54 of the first layer 30, the first 51 and second 52 lattice having same periodicity but different orientations. In the figure, grain boundaries between the different regions have been marked with lines, it should be understood that these lines are for illustrative purposes only.

Fig. 14 illustrates a perspective view of a cross-section of a filter 1 wherein the pores 32 of the first layer 30 comprise a plurality of slits 60, and wherein each slit of the plurality of slits 60 extends along a curved path across a surface of the first layer 30. A close-up view of the slit pores 32, 50 is also presented in Fig. 14.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A method (100) for producing a blood filter (1), the blood filter (1) being a filter for filtering blood, the method (100) comprising
providing (S102) a first layer (30);
providing (S104) a block copolymer layer (40) above the first layer (30);
converting (S106) the block copolymer layer (40) to a mask (48) by selectively removing domains of the block copolymer layer (40);
etching (S108) pores (32) through the first layer (30) in regions exposed by the mask (48);
wherein a pore size is below 20 nm, the pore size preferably being 6.6 nm or smaller.

2. The method (100) according to claim 1, further comprising
providing (S101) a substrate (10) before providing the first layer (30), such that the first layer (30) is provided above the substrate (10);
forming (S114) a channel (20) through the substrate (10), the channel (20) being configured to provide fluid communication between a first (21) and a second end (22) of the channel (20), the first end (21) of the channel (20) being directly below the etched pores (32) of the first layer (30),
whereby components of blood pass both the pores (32) and the channel (20), when the blood filter (1) is in use.

3. The method (100) according to claim 2, wherein
the channel (20) extends through the substrate (10) in a direction orthogonal to the first layer (30), and wherein the second end (22) of the channel (20) is arranged at a bottom side of the substrate (10); or
the channel (20) extends along an interface between the substrate (10) and the first layer (30), and wherein the second end (22) of the channel (20) is arranged at an opening (26) in the first layer (30).

4. The method (100) according to any one of the preceding claims, further comprising:
removing (S111) the mask (48); and
forming (S113) a polymer brush layer (70) above the first layer (30) after removal of the mask (48), the polymer brush layer (70) being a polymer layer comprising a plurality of polymer chains (72) wherein each polymer chain (72) is attached to an underlying layer at one end of the polymer chain (72).

5. The method (100) according to claim 4, further comprising:
providing (S103) an oxide layer (74) above the first layer (30) before providing (S104) the block copolymer layer (40); and
wherein the polymer brush layer (70) is formed above the oxide layer (74) such that the polymer chains (72) of the polymer brush layer (70) are attached to the first layer (30) by the oxide layer (74).

6. The method (100) according to any one of the preceding claims, further comprising:
depositing (S115) a film (80) on inner surfaces of the pores (32) of the first layer (30); or
oxidizing (S117) inner surfaces of the pores (32) of the first layer (30).

7. The method (100) according to any one of the preceding claims,
wherein the block copolymer layer (40) comprises vertical cylindrical domains (45), each vertical cylindrical domain (45) being a cylindrical domain with an orientation perpendicular to the first layer (30); and
wherein the block copolymer layer (40) is converted to a mask (48) by selectively removing the vertical cylindrical domains (45) of the block copolymer layer (40),
whereby the pores (32) of the first layer (30) comprise a plurality of round holes (50).

8. The method (100) according to any one of claims 1-6,
wherein the block copolymer layer (40) comprises lamella domains (46) or horizontal cylindrical domains, each horizontal cylindrical domain being a cylindrical domain with an orientation parallel with the first layer (30); and
wherein the block copolymer layer (40) is converted to a mask (48) by selectively removing the lamella domains (46) or horizontal cylindrical domains of the block copolymer layer (40),
whereby the pores (32) of the first layer (30) comprise a plurality of slits (60).

9. The method (100) according to any one of the preceding claims, the method (100) further comprising
shaping the pores (32) of the first layer (30) such that the pores (32) taper.

10. The method (100) according to any one of the preceding claims,
wherein a fill-factor of the pores (32) is at least 40%, the fill-factor of the pores (32) being open pore area per area unit of the first layer (30).

11. The method (100) according to any one of the preceding claims,
wherein the first layer (30) is 20 nm to 2000 nm thick.

12. The method (100) according to any one of the preceding claims, wherein the substrate (10) comprises one or more integrated circuits.

13. The method (100) according to any one of the preceding claims,
wherein the block copolymer layer (40) comprises:
polystyrene-polymethylmethacrylate block copolymers, and/or
polylactic acid-polyvinylpyridine block copolymers, and/or
polyethylene oxide-polydimethylsiloxane block copolymers.

14. The method (100) according to any one of the preceding claims,
wherein the block copolymer layer (40) comprises block copolymers with a molecular weight below 30 kg/mol.

15. A blood filter (1) for filtering blood, the blood filter (1) comprising:
a first layer (30);
pores (32) extending through the first layer (30), wherein a pore size is below 20 nm, the pore size preferably being 6.6 nm;
wherein the pores (32) of the first layer (30) comprises a plurality of round holes (50) and wherein the plurality of round holes (50) are arranged as a first lattice in a first region of the first layer (30) and as a second lattice in a second region of the first layer (30), the first and second lattice having same periodicity but different orientations, or
wherein the pores (32) of the first layer (30) comprises a plurality of slits (60), and wherein each slit of the plurality of slits (60) extends along a curved path across a surface of the first layer (30).
